# EUROPEAN PATENT SPECIFICATION

(11) **EP 4 493 254 B1**
(45) Date of publication and mention of the grant of the patent: **29.04.2026**
(21) Application number: 22715012.5
(22) Date of filing: 14.03.2022
(51) Int. Cl.: A61M 25/10, A61F 2/00, A61F 2/958

(54) **A BALLOON PROTECTOR FOR A BALLOON CATHETER**
BALLONSCHUTZ FÜR EINEN BALLONKATHETER
PROTECTEUR DE BALLONNET POUR CATHÉTER À BALLONNET

(43) Date of publication of application: 22.01.2025
(73) Proprietor: Clearstream Technologies Limited, Enniscorthy, County Wexford (IE)
(72) Inventor: GRIFFIN, Piaras, Enniscorthy, County Wexford (IE); RONAN, Allan, Enniscorthy, County Wexford (IE); SPODOBALSKI, Artur, Enniscorthy, County Wexford (IE)
(74) Representative: Hoffmann Eitle
(86) International application number: PCT/EP2022/056514
(87) International publication number: WO 2023/174507

(56) References cited:
- EP-B1- 0 973 576
- US-A- 5 053 007
- US-A1- 2004 093 005
- US-A1- 2015 217 092

## Description

### Technical Field

The present disclosure relates to a balloon protector for a balloon catheter; a kit comprising a balloon protector and a balloon catheter; and a method of protecting a balloon of a balloon catheter with a balloon protector.

### Background

Angioplasty is a procedure used for the treatment of blockages or stenosis in blood vessels, e.g. arteries. Blockages may occur from cholesterol build up on blood vessel walls or due to formation of thrombus. In angioplasty procedures, a dilatation balloon catheter is generally used in an effort to dilate the blood vessel and open up the blockage area. A balloon catheter may be inserted into a blood vessel of a patient using an introducer. The balloon catheter may be inserted through the introducer and advanced through a blood vessel until the distal end of the balloon catheter is at a desired location in the vasculature, e.g. at the site of a blockage or stenosis. A guide wire may be introduced and used to guide the balloon catheter to the desired location. The balloon catheter is advanced over the guide wire until the balloon is properly positioned. Once properly positioned in a blockage or stenosis area, an expandable balloon at the distal end of the balloon catheter may be inflated, e.g. by passing a fluid through an inflation lumen into the balloon. The fluid pressure radially expands the balloon which dilates the lumen of the blood vessel and compresses the plaque of the blockage or stenosis.

To perform angioplasty procedures, it is desirable for the balloon catheter to have a narrow profile, or relatively small deflated cross-sectional diameter so it is easier to advance the balloon catheter into a stenosis or blockage area. The balloon of the angioplasty catheter is often formed from a very thin polymeric material to provide for a narrower profile. Further, the balloon can be wrapped or folded about the shaft of the catheter into a tightly folded, deflated configuration, which helps to minimize the profile. While balloons are generally capable of developing high pressures under inflation, the balloons are delicate and can be damaged such that the balloon may fail during inflation. For example, the material of the balloons may be susceptible to scratches or other damage, e.g. during shipping and/or handling, which can result in premature balloon failure. It may also be desirable to coat balloons for use in angioplasty for various purposes. For example, it may be desirable to coat balloons with bioactive agents or drugs. For example, anti-restenosis, anti-coagulent, and/or anti-thrombogenic drugs coated on an angioplasty balloon may help prevent restenosis. These coatings must also be protected to prevent these coatings from being scraped off or diminished.

Accordingly, it is desirable to protect the balloon from damage until it is used. A balloon protector in the form of a protective sheath or sleeve may be applied over the balloon to provide this protection. The protective sheath also helps to maintain the balloon in its tightly folded, low profile configuration during shipping, handling, and storage. Examples of balloon protector sleeves are disclosed in US 5,893,868 and US 5,015,231. Another example of a balloon protector can be found in EP 0 973 576.

However, these balloon protector sleeves can be difficult to apply to and remove from the balloon without damaging the balloon or removing the coating. Furthermore, these balloon protector sleeves are limited by the amount they can reduce the profile of the balloon.

In view of this, there is hence a need in the art for a balloon protector which can be easily applied to and removed from the balloon without damaging the balloon or removing the coating.

There is further a need in the art for a balloon protector which can reduce the profile of the balloon to improve trackability of the balloon catheter.

There is further a need in the art for a balloon protector which provides improved trackability and flexibility of the balloon catheter.

### Summary

The invention is defined in the claims.

In a first aspect of the present disclosure, there is provided a balloon protector for a balloon catheter. The balloon protector comprises an inner sleeve having an interior for receiving a balloon and a tapered outer surface. The balloon catheter further comprises an outer sleeve having a tapered inner surface. The interior of the inner sleeve has an expanded configuration and a contracted configuration. The tapered outer surface of the inner sleeve engages the tapered inner surface of the outer sleeve such that pushing the inner sleeve and the outer sleeve will move the interior of the inner sleeve from the expanded configuration to the contracted configuration. The interior of the inner sleeve is cylindrical in the expanded configuration.

In some embodiments, this may result in a balloon protector which can more effectively decrease the profile of the balloon to improve trackability of the balloon catheter.

In some embodiments, this may further allow the balloon protector to be easily applied to and removed from the balloon without damaging the balloon or removing the coating. Throughout this disclosure, the term 'expanded configuration' is used to denote a configuration of an element in which the diameter of the element is greater than in a 'collapsed configuration'.

The inner sleeve and outer sleeve may be pushed together in a longitudinal direction in order to move the interior of the inner sleeve from the expanded configuration to the contracted configuration.

The inner sleeve may have a proximal and distal opening.

In some embodiments, this may allow a protective shipping mandrel to be more easily disposed within the balloon catheter.

The outer sleeve may have a proximal and distal opening.

In some embodiments, this may allow a protective shipping mandrel to be more easily disposed within the balloon catheter.

Throughout this disclosure, the term 'cylindrical' will be used within its normal meaning of having straight parallel sides and a circular or oval cross section, i.e. the sides are not tapered.

The interior of the inner sleeve may be cylindrical in the contracted configuration.

In some embodiments, this may apply an even pressure on the balloon to provide a more even reduced profile of the balloon and ensure better trackability.

The inner sleeve may comprise one or more slots.

In some embodiments, this may allow the diameter of the inner sleeve to more effectively decrease due to radial forces exerted from pushing the outer sleeve over the inner sleeve

The one or more slots may be longitudinal slots.

The one or more slots may be helical slots.

The balloon protector may further comprise at least one checkering element disposed on an inner surface of the inner sleeve, for forming one or more indentations in the surface of the balloon.

In some embodiments, this may provide improved flexibility and trackability to the balloon catheter.

In some embodiments, this may further reduce the chance of the balloon protector slipping off the balloon.

The at least one checkering element may be moulded to the inner sleeve.

In some embodiments, this may result in a more secure fit between the checkering element and inner sleeve.

The at least one checkering element may comprise one or more rings.

The one or more rings may be longitudinally spaced.

The one or more rings may be circumferential rings.

The at least one checkering element may comprise a helical element.

The inner sleeve and the outer sleeve may be made from a polymer material.

In some embodiments, this may result in a light, portable and durable balloon protector.

The outer sleeve may be made from a harder material than the inner sleeve.

In some embodiments, this may provide protection to the balloon whilst also effectively decreasing the profile of the balloon to improve trackability of the balloon catheter.

In a second aspect of the present disclosure, there is provided a kit. The kit comprises the balloon protector according to any of the above statements. The kit further comprises a balloon catheter comprising a shaft and a balloon disposed at the distal end of the shaft. The balloon is positioned within the interior of the inner sleeve.

In some embodiments, this may result in kit having a balloon protector which can be easily applied to and removed from the balloon without damaging the balloon or removing the coating and also effectively decrease the profile of the balloon to improve trackability of the balloon catheter.

The surface of the balloon may be coated with a drug.

In some embodiments, this may result in a kit which can protect the balloon without removing the coating.

The balloon may be folded.

In the contracted configuration, the inner surface of the inner sleeve may be in contact with the balloon.

The kit may further comprise a protection mandrel positioned within the distal end of the balloon catheter.

In some embodiments, this may better protect the balloon catheter during shipping, transport and handling.

In a third aspect of the present disclosure, there is provided a method of protecting a balloon of a balloon catheter with a balloon protector having an inner sleeve with a tapered outer surface and an outer sleeve with a tapered inner surface. The method comprises inserting the balloon into an interior of the inner sleeve and reducing the profile of the balloon by pushing the inner sleeve and the outer sleeve together to move the interior of the inner sleeve from an expanded configuration to a contracted configuration. The interior of the inner sleeve is cylindrical in the expanded configuration.

In some embodiments, this method may allow easy application and removal of the balloon protector from the balloon without damaging the balloon or removing the coating and also allows the profile of the balloon to be effectively decreased to improve trackability of the balloon catheter.

The method may further comprise forming one or more indentations in the surface of the balloon with at least one checkering element disposed on an inner surface of the inner sleeve.

In some embodiments, this method may allow the flexibility and trackability of the balloon catheter to be improved.

The one or more indentations may be ring-like indentations formed by one or more checkering rings.

The one or more indentations may be helical indentations formed by one or more helical checkering elements.

### Brief Description of the Drawings

To enable better understanding of the present disclosure, and to show how the same may be carried into effect, reference will now be made, by way of example only, to the accompanying drawings, in which:
FIG. 1A shows a cross-sectional side view of a balloon catheter disposed within a balloon protector in the expanded configuration according to the present disclosure.
FIG. 1B shows a cross-sectional side view of a balloon catheter disposed within the balloon protector of FIG. 1A in the contracted configuration.
FIGS. 2A and 2B show side views of two different embodiments of an inner sleeve of a balloon protector according to the present disclosure.
FIG. 3A shows a cross-sectional side view of a balloon catheter disposed within an alternative balloon protector in the expanded configuration according to the present disclosure.
FIG. 3B shows a cross-sectional side view of a balloon catheter disposed within the balloon protector of FIG. 3A in the contracted configuration.

### Detailed Description

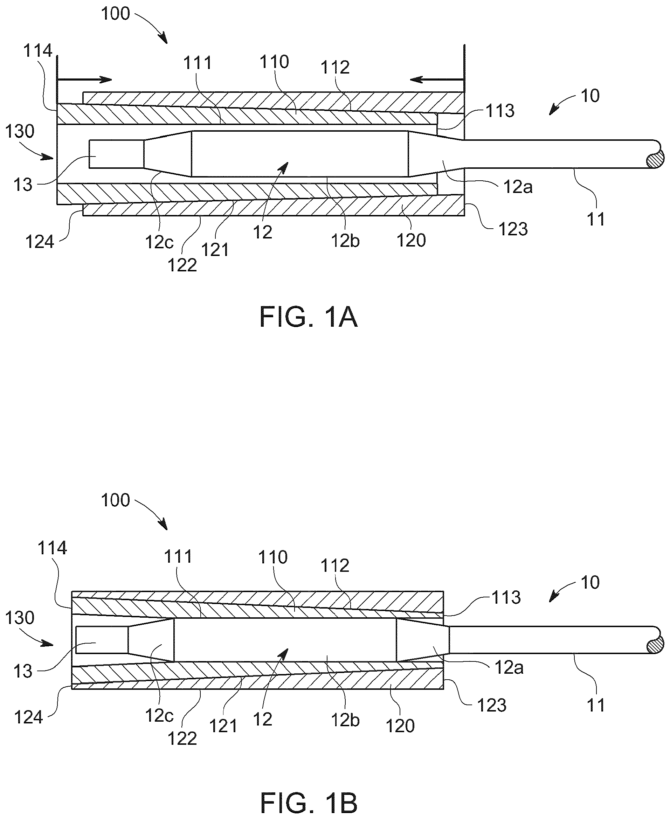

FIG. 1A shows a balloon catheter 10 disposed within a balloon protector 100.

The balloon catheter 10 may be a standard dilatation catheter for performing balloon angioplasty procedures. The balloon catheter 10 comprises a catheter shaft 11, a balloon 12 disposed at the distal end of the catheter shaft 11, and a distal tip 13. The balloon 12 may have a proximal tapered portion 12a, a central barrel portion 12b and a distal tapered portion 12c. The balloon 12 may be a compliant, semi-compliant or non-compliant balloon and made from any suitable material, such as, for example, PVC, PET, nylon, polyester, PEBAX, polyurethane or silicone.

The balloon protector 100 is shown in an expanded configuration and comprises an inner sleeve 110 and an outer sleeve 120. The inner sleeve 110 has an inner surface 111 which defines an interior 130 of the balloon protector 100 where the balloon 12 is accommodated. In the expanded configuration, as shown in FIG. 1A, the interior 130 of the inner sleeve 110 has a greater diameter than it does in a contracted configuration. The inner sleeve 110 further has an outer surface 112 which is a tapered surface. The diameter of the outer surface 112 is smaller at a first end 113, for example a proximal end, than it is at an opposite second end 114, for example a distal end.

The interior 130 of the balloon protector 110, defined by the inner surface 111, is cylindrical in the expanded configuration. This allows the balloon 12 to be easily inserted into the interior 130 without rubbing or scraping the outside surface of the balloon and thereby prevents damaging the balloon 12 or removing coating which may be present on the surface of the balloon.

The outer sleeve 120 has an inner surface 121 which is tapered and engages with the tapered outer surface 112 of the inner sleeve 110. The inner surface 121 may be tapered in the same direction to the outer surface 112 of the inner sleeve 110, such that the diameter of the inner surface 121 is smaller at a first end 113, for example a proximal end, than it is at the opposite second end 114, for example a distal end. The outer sleeve further has an outer surface 122 which may be a non-tapered cylindrical surface.

The inner sleeve 110 and the outer sleeve 120 may be made from a polymer material, such as PTFE, nylon, Pebax or polyurethane. The inner and outer sleeves 110, 120 may be made from the same material. Alternatively, the outer sleeve 120 may be made from a harder material than the inner sleeve 110. For example, the outer sleeve may be made from 'nylon 12' while the inner sleeve 110 may be made from Pebax. The harder outer sleeve 120 can better protect the balloon 12 from damage, while the more flexible inner sleeve 110 can expand and contract to allow easier movement between the expanded and contracted configuration.

Both the inner sleeve 110 and outer sleeve 120 have an opening at the proximal end 113, 123 to allow insertion of the balloon 12. The inner sleeve 110 and outer sleeve 120 may further have an opening at the distal end 114, 124 to allow for the insertion of a shipping mandrel which may protect the balloon catheter 10 during shipping and transport.

FIG. 1B shows the balloon catheter 10 disposed within the balloon protector 100, with the balloon catheter 100 in a contracted configuration. In the contracted configuration, the diameter of the interior 130 is smaller than in the expanded configuration. The interior 130 may maintain a cylindrical shape in the contracted configuration, which helps to apply an even and constant circumferential pressure to the balloon 12.

In order to move the balloon protector 100 from the expanded configuration to the contracted configuration, a user can push the inner sleeve 110 and the outer sleeve 120 together in a longitudinal direction (see the arrows shown in FIG. 1A). The engagement of the tapered inner surface 121 of the outer sleeve 120 with the tapered outer surface 112 of the inner sleeve 110 will cause the inner sleeve 110 to contract and thereby reduce the diameter of the interior 130 of the balloon protector 100.

In order to apply the balloon protector 100 to the balloon catheter 10, the balloon 12 of the balloon catheter 10 is first inserted into the interior 130 of the balloon protector 100 in the expanded configuration. The balloon 12 may be folded to reduce the profile. The balloon 12 and the balloon protector 100 are sized such that the diameter of the inner surface 111 is greater than the diameter of the balloon 12. This allows the balloon 12 to be easily inserted into the interior 130 of the balloon protector 100, without excessive friction and prevents the balloon 12 being damaged or the coating, if present, from being rubbed off.

Once the balloon is positioned within the interior 130 of the balloon protector 100 in the expanded configuration, as shown in FIG. 1A, the inner sleeve 110 and the outer sleeve 120 are pushed together to collapse the interior 130 of the balloon protector 100 from the expanded configuration to the contracted configuration. This causes the diameter of the interior 130 to reduce such that the inner surface 111 comes into contact with the balloon 12 and applies pressure to the balloon 12, as shown in FIG. 1B. This allows the profile of the balloon 12 to be effectively decreased to improves trackability of the balloon catheter 10.

A shipping mandrel can then be inserted into the distal end 13 of the balloon catheter 10 to further protect the balloon catheter 10 during shipping and transport.

When the balloon catheter 10 is to be used, for example in an angioplasty procedure, the balloon catheter 10 can be easily removed from the balloon protector 100 without damaging the balloon 12 or removing the coating which may be present on the balloon 12. To remove the balloon catheter 10, the inner sleeve 110 and outer sleeve 120 can simply be pulled apart in a longitudinal direction. This results in balloon protector 100 moving from the contracted configuration to the expanded configuration and the diameter of the interior 130 increasing. The balloon 12 can then be removed from the balloon protector 100 with little to no force thus negating the risk of damaging the balloon 12 due to pulling it out of a tight sleeve. Furthermore, this also avoids the unnecessary loss of any drugs which may be coated on the surface of the balloon 12. Due to the pressure applied by the balloon protector 100 on the balloon 12, the profile of the balloon 12 is reduced which will result in increased trackability of the balloon catheter when it is inserted into a patient to perform an angioplasty procedure, for example.

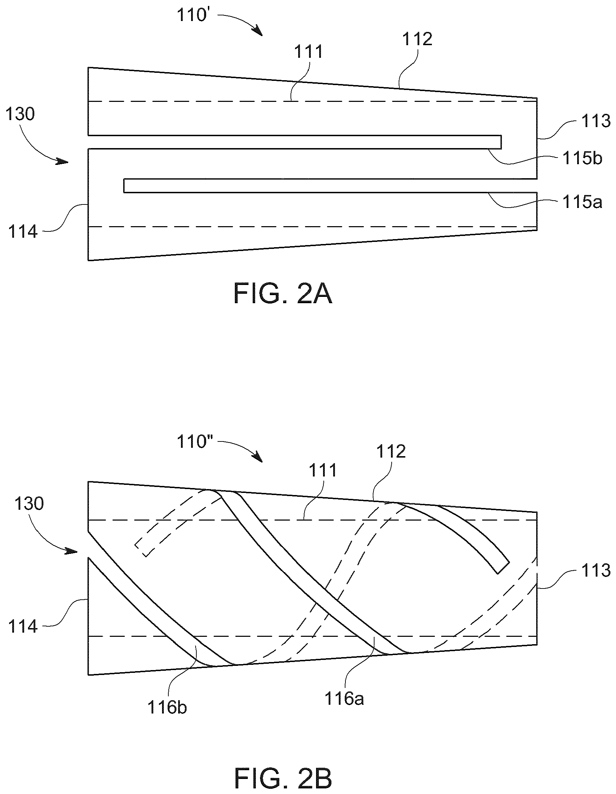

In some cases, the inner sleeve 110 may be provided with slots to allow easier expansion and contraction of the inner sleeve 110 when moving between the contracted and expanded configuration. FIG. 2A shows a side view of an alternative embodiment of an inner sleeve 110'. The same reference numerals will be used throughout this disclosure for features which are identical across different embodiments.

The inner sleeve 110' comprises an inner surface 111 and a tapered outer surface 112. The inner surface 111 defines an interior 130 of the inner sleeve 110' which may be cylindrical in shape. The inner sleeve 110' further comprises a first longitudinal slot 115a and a second longitudinal slot 115b. The longitudinal slots 115a,b increase the flexibility of the inner sleeve 110' such that it can contract and expand more easily. The first longitudinal slot 115a may be open to a first end 113 of the inner sleeve 110', while the second longitudinal slot may be open to a second end 114 of the inner sleeve 110'. Having two longitudinal slots which are open on opposite ends of the inner sleeve 110' helps to maintain the interior 130 of the inner sleeve 110' in a cylindrical shape when moving between the contracted and expanded configuration. This way, the inner sleeve 110' can apply an even and constant circumferential pressure on the balloon 12 in the contracted configuration.

FIG. 2B shows a side view of an alternative inner sleeve 110" having an inner surface 111 and a tapered outer surface 112. The inner sleeve 110" further comprises a first helical slot 116a and a second helical slot 116b. The first helical slot 116a is open to a first end 113 of the inner sleeve 110' while the second helical slot 116b is open to a second end 114 of the inner sleeve 110'.

The helical slots 116 serve the same function as the longitudinal slots 115 of FIG. 2A in that they allow the inner sleeve 110' to more easily expand and contract whilst maintaining a cylindrical shaped interior 130.

Both the inner sleeves 110' and 110'' can be used with the balloon protector 100 described with respect to FIG. 1A and B, above.

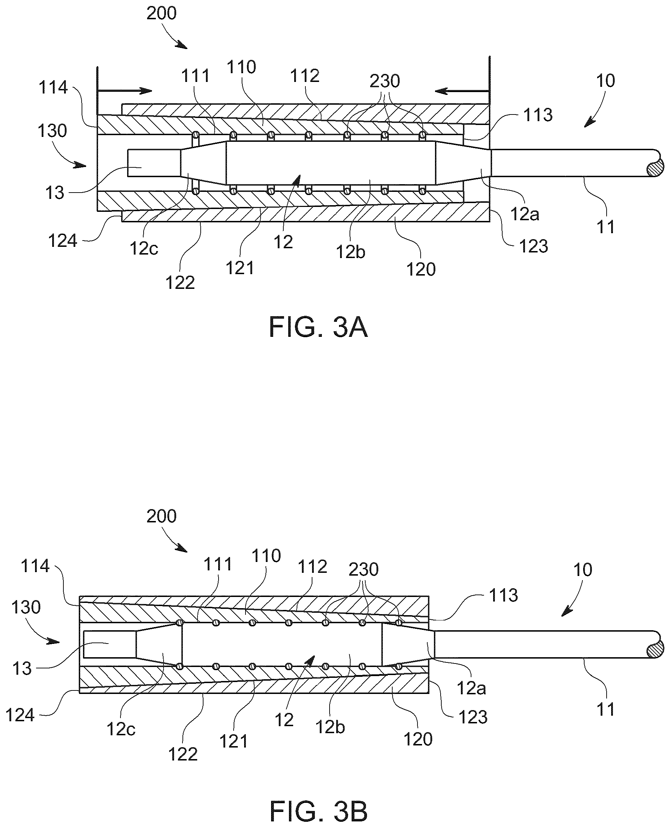

FIG. 3A shows the balloon catheter 10 and an alternative embodiment of a balloon protector 200 in an expanded configuration. The balloon protector 200 is identical to balloon protector 100 in many ways and the features which are the same across the embodiments are marked with the same reference numerals.

Balloon protector 200 differs from balloon protector 100 in that it further comprises one or more checkering elements 230 which are attached to the inner surface 111 of the inner sleeve 110. The checkering elements 230 are shown in FIG. 3A as a number of circumferential rings which are longitudinally spaced along the length of the inner surface 111 of the inner sleeve 110, but may also be in the form of a helical spiral, for example. The checkering elements 230 may be attached to the inner sleeve 110, for example, by moulding them to the inner surface 111 which ensures a secure fit. The checkering elements 230 may be made from the same material as inner sleeve 110 above, e.g. PTFE, nylon, Pebax or polyurethane, or any other suitable material, such as stainless steel or nitinol.

FIG. 2B shows the balloon catheter 10 disposed within the balloon protector 200 in the contracted configuration.

The balloon protector 200 can be moved from the expanded configuration to the contracted configuration in the same manner as balloon protector 100 by pushing together the inner sleeve 110 and the outer sleeve 120 in a longitudinal direction. In the contracted configuration, the checkering elements 230 are pressed into the balloon 12 and especially the barrel portion 12b. The checkering element 230 leave marks on the balloon which act as flexi-points about which the barrel portion 12b of the balloon 12 can more easily bend. The flexibility and trackability of the balloon catheter 10 are therefore improved, which allows the balloon catheter 10 to be more effectively delivered to a treatment site in the human body when performing an angioplasty procedure, for example.

The balloon protector 200 may equally have an inner sleeve 110 comprising slots, such as inner sleeves 110' or 110". The method of applying and removing the balloon protector 200 from the balloon catheter 10 is the same as described above for balloon catheter 100.

Various modifications will be apparent to those skilled in the art.

The interior 130 of the balloon protector 100, 200 may not be cylindrical but may take another suitable shape and may have a taper, for example. In the present invention, the interior of the inner sleeve is cylindrical in the expanded configuration.

The inner sleeve 110 and the outer sleeve 120 may each comprise a handle element, for example, to facilitate pushing together and pulling apart of the inner and outer sleeve 110, 120.

The outer surface 122 of the outer sleeve 120 is not limited to any specific type of shape.

The inner sleeve 110 and the outer sleeve 120 are not limited to any type of material but may be made from any suitable material.

The inner sleeve 110 and the outer sleeve 120 may not have a proximal opening or a distal opening.

The inner sleeve 110' is not limited to having two longitudinal slots 115. The inner sleeve 110' may comprise fewer or more longitudinal slots. For example, the inner sleeve 110' may only have one longitudinal slot which extends along the entire length of the sleeve 110' and is open to both the first end 113 and the second end 114. Alternatively, the inner sleeve 110' may comprise more than two longitudinal slots.

The inner sleeve 110' is not limited to having two helical slots 116. For example, the inner sleeve 110' may comprise only one helical slot which extends along the length of the inner sleeve 110" and is open to both ends 113, 114. Alternatively, the inner sleeve 110" may also comprise more than two helical slots.

The checkering elements 230 are not limited to being in the form of circumferential rings. The checkering elements may take any other suitable shape such as, for example, a helical spiral, C-shaped elements or bumps.

All of the above are fully within the scope of the present disclosure and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such modifications or adaptations fall within the scope of the present disclosure.

## Claims

1. A balloon protector (100) for a balloon catheter (10), the balloon protector comprising:
an inner sleeve (110) having an interior (130) for receiving a balloon (12) and a tapered outer surface (112);
an outer sleeve (120) having a tapered inner surface (121),
wherein the interior (130) of the inner sleeve (110) has an expanded configuration and a contracted configuration, and
wherein the tapered outer surface (112) of the inner sleeve (110) engages the tapered inner surface (121) of the outer sleeve (120) such that pushing the inner sleeve (110) and the outer sleeve (120) together will move the interior (130) of the inner sleeve (110) from the expanded configuration to the contracted configuration,
**characterised in that**
the interior (130) of the inner sleeve (110) is cylindrical in the expanded configuration.

2. The balloon protector of claim 1, wherein the inner sleeve (110) has a proximal and distal opening, and/or wherein the outer sleeve (120) has a proximal and distal opening.

3. The balloon protector of any preceding claim, wherein the interior (130) of the inner sleeve (110) is cylindrical in the contracted configuration.

4. The balloon protector of any preceding claim, wherein the tapered inner surface (121) of the outer sleeve (120) is tapered in the same direction as the tapered outer surface (112) of the inner sleeve (110) such that the diameter of the tapered inner surface (121) is smaller at a first end (113) of the outer sleeve than it is at a second end (114) of the outer sleeve.

5. The balloon protector of any preceding claim, wherein the inner sleeve (110) comprises one or more slots (115, 116), optionally
wherein the one or more slots are longitudinal slots (115), or
wherein the one or more slots are helical slots (116).

6. The balloon protector of any preceding claim, further comprising at least one checkering element (230) disposed on an inner surface (111) of the inner sleeve (110), for forming one or more indentations in the surface of the balloon (12), optionally
wherein the at least one checkering element (130) is moulded to the inner sleeve (110).

7. The balloon protector of claim 6, wherein the at least one checkering element (230) comprises one or more rings, optionally
wherein the one or more rings are longitudinally spaced, and/or
wherein the one or more rings are circumferential rings.

8. The balloon protector of claim 6, wherein the at least one checkering element (230) comprises a helical element.

9. The balloon protector of any preceding claim, wherein the inner sleeve (110) and the outer sleeve (120) are made from a polymer material, and/or
wherein the outer sleeve (120) is made from a harder material than the inner sleeve (110).

10. A kit comprising:
the balloon protector (100) of any preceding claim;
a balloon catheter (10) comprising a shaft (11) and a balloon (12) disposed at the distal end of the shaft (11),
wherein the balloon (12) is positioned within the interior (130) of the inner sleeve (110).

11. The kit of claim 10, wherein the surface of the balloon (12) is coated with a drug, and/or
wherein the balloon (12) is folded.

12. The kit of any of claims 10 to 11, wherein in the contracted configuration, the inner surface (111) of the inner sleeve (110) is in contact with the balloon (12), and/or
further comprising a protection mandrel positioned within the distal end of the balloon catheter.

13. A method of protecting a balloon (12) of a balloon catheter (10) with a balloon protector (100) having an inner sleeve (110) with a tapered outer surface (112) and an outer sleeve (120) with a tapered inner surface (121), the method comprising:
inserting the balloon (12) into an interior (130) of the inner sleeve (110);
reducing the profile of the balloon (12) by pushing the inner sleeve (110) and the outer sleeve (120) together to move the interior (130) of the inner sleeve (110) from an expanded configuration to a contracted configuration,
**characterized in that**
the interior (130) of the inner sleeve is cylindrical in the expanded configuration.

14. The method of claim 13, further comprising forming one or more indentations in the surface of the balloon (12) with at least one checkering element (230) disposed on an inner surface (112) of the inner sleeve (110), and/or
wherein the tapered inner surface (121) of the outer sleeve (120) is tapered in the same direction as the tapered outer surface (112) of the inner sleeve (110) such that the diameter of the tapered inner surface (121) is smaller at a first end (113) of the outer sleeve (120) than it is at a second end (114) of the outer sleeve (120).

15. The method of claim 14, wherein the one or more indentations are ring-like indentations formed by one or more checkering rings (230), or
wherein the one or more indentations are helical indentations formed by one or more helical checkering elements (230).

## Patentansprüche

1. Ballonschutz (100) für einen Ballonkatheter (10), wobei der Ballonschutz umfasst:
eine Innenhülse (110), die einen Innenraum (130) zur Aufnahme eines Ballons (12) und eine konische Außenfläche (112) aufweist;
eine Außenhülse (120), die eine konische Innenfläche (121) aufweist,
wobei der Innenraum (130) der Innenhülse (110) eine ausgedehnte Konfiguration und eine zusammengezogene Konfiguration aufweist, und
wobei die konische Außenfläche (112) der Innenhülse (110) in die konische Innenfläche (121) der Außenhülse (120) eingreift, so dass das Zusammenschieben der Innenhülse (110) und der Außenhülse (120) den Innenraum (130) der Innenhülse (110) von der ausgedehnten Konfiguration in die zusammengezogene Konfiguration bewegt,
**dadurch gekennzeichnet ist, dass**
der Innenraum (130) der Innenhülse (110) in der ausgedehnten Konfiguration zylindrisch ist.

2. Ballonschutz nach Anspruch 1, wobei die Innenhülse (110) eine proximale und distale Öffnung aufweist, und/oder wobei die Außenhülse (120) eine proximale und distale Öffnung aufweist.

3. Ballonschutz nach einem vorstehenden Anspruch, wobei der Innenraum (130) der Innenhülse (110) in der zusammengezogenen Konfiguration zylindrisch ist.

4. Ballonschutz nach einem vorstehenden Anspruch, wobei die konische Innenfläche (121) der Außenhülse (120) in die gleiche Richtung wie die konische Außenfläche (112) der Innenhülse (110) konisch ist, so dass der Durchmesser der konischen Innenfläche (121) an einem ersten Ende (113) der Außenhülse kleiner ist als an einem zweiten Ende (114) der Außenhülse.

5. Ballonschutz nach einem vorstehenden Anspruch, wobei die Innenhülse (110) einen oder mehrere Schlitze (115, 116) umfasst, wahlweise
wobei der eine oder die mehreren Schlitze Längsschlitze (115) sind, oder
wobei der eine oder die mehreren Schlitze spiralförmige Schlitze (116) sind.

6. Ballonschutz nach einem vorstehenden Anspruch, weiter umfassend mindestens ein Rändelungselement (230), das auf einer Innenfläche (111) der Innenhülse (110) angeordnet ist, um eine oder mehrere Vertiefungen in der Oberfläche des Ballons (12) zu bilden, wahlweise
wobei das mindestens eine Rändelungselement (130) an die Innenhülse (110) aufgeformt ist.

7. Ballonschutz nach Anspruch 6, wobei das mindestens eine Rändelungselement (230) einen oder mehrere Ringe umfasst, wahlweise
wobei der eine oder die mehreren Ringe längs beabstandet sind, und/oder
wobei der eine oder die mehreren Ringe Umfangsringe sind.

8. Ballonschutz nach Anspruch 6, wobei das mindestens eine Rändelungselement (230) ein spiralförmiges Element umfasst.

9. Ballonschutz nach einem vorstehenden Anspruch, wobei die Innenhülse (110) und die Außenhülse (120) aus einem Polymermaterial hergestellt sind, und/oder
wobei die Außenhülse (120) aus einem härteren Material als die Innenhülse (110) hergestellt sind.

10. Kit, umfassend:
den Ballonschutz (100) nach einem vorstehenden Anspruch;
einen Ballonkatheter (10), der einen Schaft (11) und einen Ballon (12) umfasst, der an dem distalen Ende des Schafts (11) angeordnet ist,
wobei der Ballon (12) innerhalb des Innenraums (130) der Innenhülse (110) positioniert ist.

11. Kit nach Anspruch 10, wobei die Oberfläche des Ballons (12) mit einem Medikament beschichtet ist, und/oder
wobei der Ballon (12) gefaltet ist.

12. Kit nach einem der Ansprüche 10 bis 11, wobei in der zusammengezogenen Konfiguration die Innenfläche (111) der Innenhülse (110) in Kontakt mit dem Ballon (12) ist, und/oder
weiter umfassend einen Schutzdorn, der innerhalb des distalen Endes des Ballonkatheters positioniert ist.

13. Verfahren zum Schutz eines Ballons (12) eines Ballonkatheters (10) mit einem Ballonschutz (100), der eine Innenhülse (110) mit einer konischen Außenfläche (112) und eine Außenhülse (120) mit einer konischen Innenfläche (121) aufweist, wobei das Verfahren umfasst:
Einführen des Ballons (12) in einen Innenraum (130) der Innenhülse (110);
Reduzieren des Profils des Ballons (12) durch Zusammenschieben der Innenhülse (110) und der Außenhülse (120), um den Innenraum (130) der Innenhülse (110) von einer ausgedehnten Konfiguration in eine zusammengezogene Konfiguration zu bewegen,
**dadurch gekennzeichnet, dass**
der Innenraum (130) der Innenhülse in der ausgedehnten Konfiguration zylindrisch ist.

14. Verfahren nach Anspruch 13, weiter umfassend das Bilden einer oder mehrerer Vertiefungen in der Oberfläche des Ballons (12) mit mindestens einem Rändelungselement (230), das auf einer Innenfläche (112) der Innenhülse (110) angeordnet ist, und/oder
wobei die konische Innenfläche (121) der Außenhülse (120) in die gleiche Richtung wie die konische Außenfläche (112) der Innenhülse (110) konisch ist, so dass der Durchmesser der konischen Innenfläche (121) an einem ersten Ende (113) der Außenhülse (120) kleiner ist als an einem zweiten Ende (114) der Außenhülse (120).

15. Verfahren nach Anspruch 14, wobei die eine oder die mehreren Vertiefungen ringförmige Vertiefungen sind, die durch einen oder mehrere Rändelungsringe (230) gebildet werden, oder
wobei die eine oder die mehreren Vertiefungen spiralförmige Vertiefungen sind, die durch ein oder mehrere spiralförmige Rändelungselemente (230) gebildet werden.

## Revendications

1. Protecteur (100) de ballonnet pour un cathéter (10) à ballonnet, le protecteur de ballonnet comprenant :
un manchon interne (110) présentant un intérieur (130) pour recevoir un ballonnet (12) et une surface externe conique (112) ;
un manchon externe (120) présentant une surface interne conique (121),
dans lequel l'intérieur (130) du manchon interne (110) présente une configuration expansée et une configuration contractée, et
dans lequel la surface externe conique (112) du manchon interne (110) vient en prise avec la surface interne conique (121) du manchon externe (120) de sorte qu'une poussée du manchon interne (110) et du manchon externe (120) ensemble déplacera l'intérieur (130) du manchon interne (110) de la configuration expansée à la configuration contractée,
**caractérisé en ce que**
l'intérieur (130) du manchon interne (110) est cylindrique dans la configuration expansée.

2. Protecteur de ballonnet selon la revendication 1, dans lequel le manchon interne (110) présente une ouverture proximale et distale, et/ou dans lequel le manchon externe (120) présente une ouverture proximale et distale.

3. Protecteur de ballonnet selon une quelconque revendication précédente, dans lequel l'intérieur (130) du manchon interne (110) est cylindrique dans la configuration contractée.

4. Protecteur de ballonnet selon une quelconque revendication précédente, dans lequel la surface interne conique (121) du manchon externe (120) est conique dans la même direction que la surface externe conique (112) du manchon interne (110) de sorte que le diamètre de la surface interne conique (121) est plus petit à une première extrémité (113) du manchon externe qu'il ne l'est à une seconde extrémité (114) du manchon externe.

5. Protecteur de ballonnet selon une quelconque revendication précédente, dans lequel le manchon interne (110) comprend une ou plusieurs fentes (115, 116), facultativement
dans lequel les une ou plusieurs fentes sont des fentes (115) longitudinales, ou
dans lequel les une ou plusieurs fentes sont des fentes (116) hélicoïdales

6. Protecteur de ballonnet selon une quelconque revendication précédente, comprenant en outre au moins un élément de motif à damier (230) disposé sur une surface interne (111) du manchon interne (110), pour la formation d'une ou plusieurs indentations dans la surface du ballonnet (12), facultativement
dans lequel l'au moins un élément de motif à damier (130) est moulé sur le manchon interne (110).

7. Protecteur de ballonnet selon la revendication 6, dans lequel l'au moins un élément de motif à damier (230) comprend un ou plusieurs anneaux, facultativement
dans lequel les un ou plusieurs anneaux sont espacés dans le sens longitudinal, et/ou
dans lequel les un ou plusieurs anneaux sont des anneaux circonférentiels.

8. Protecteur de ballonnet selon la revendication 6, dans lequel l'au moins un élément de motif à damier (230) comprend un élément hélicoïdal.

9. Protecteur de ballonnet selon une quelconque revendication précédente, dans lequel le manchon interne (110) et le manchon externe (120) sont constitués d'un matériau polymère, et/ou
dans lequel le manchon externe (120) est constitué d'un matériau plus dur que le manchon interne (110).

10. Kit comprenant :
le protecteur (100) de ballonnet selon une quelconque revendication précédente ;
un cathéter (10) à ballonnet comprenant une tige (11) et un ballonnet (12) disposé à l'extrémité distale de la tige (11),
dans lequel le ballonnet (12) est positionné au sein de l'intérieur (130) du manchon interne (110).

11. Kit selon la revendication 10, dans lequel la surface du ballonnet (12) est revêtue d'un médicament, et/ou
dans lequel le ballonnet (12) est plié.

12. Kit selon l'une quelconque des revendications 10 et 11, dans lequel dans la configuration contractée, la surface interne (111) du manchon interne (110) est en contact avec le ballonnet (12),
et/ou
comprenant en outre un mandrin de protection positionné au sein de l'extrémité distale du cathéter à ballonnet.

13. Procédé de protection d'un ballonnet (12) d'un cathéter (10) à ballonnet avec un protecteur (100) de ballonnet présentant un manchon interne (110) avec une surface externe conique (112) et un manchon externe (120) avec une surface interne conique (121), le procédé comprenant :
l'insertion du ballonnet (12) dans un intérieur (130) du manchon interne (110) ;
la réduction du profil du ballonnet (12) par poussée du manchon interne (110) et du manchon externe (120) ensemble pour déplacer l'intérieur (130) du manchon interne (110) d'une configuration expansée à une configuration contractée,
**caractérisé en ce que**
l'intérieur (130) du manchon interne est cylindrique dans la configuration expansée.

14. Procédé selon la revendication 13, comprenant en outre la formation d'une ou plusieurs indentations dans la surface du ballonnet (12) avec au moins un élément de motif à damier (230) disposé sur une surface interne (112) du manchon interne (110), et/ou
dans lequel la surface interne conique (121) du manchon externe (120) est conique dans la même direction que la surface externe conique (112) du manchon interne (110) de sorte que le diamètre de la surface interne conique (121) est plus petit à une première extrémité (113) du manchon externe (120) qu'il ne l'est à une seconde extrémité (114) du manchon externe (120).

15. Procédé selon la revendication 14, dans lequel les une ou plusieurs indentations sont des indentations de type anneau formées par un ou plusieurs anneaux de motif à damier (230), ou
dans lequel les une ou plusieurs indentations sont des indentations hélicoïdales formées par un ou plusieurs éléments de motif à damier (230) hélicoïdaux.
